# EUROPEAN PATENT SPECIFICATION

(11) **EP 4 479 048 B1**
(45) Date of publication and mention of the grant of the patent: **18.03.2026**
(21) Application number: 23705558.7
(22) Date of filing: 16.02.2023
(51) Int. Cl.: A61K 31/454, A61K 9/08, A61K 47/40, A61P 37/08

(54) **BILASTINE COMPOSITION FOR ONCE-DAILY PARENTERAL ADMINISTRATION**
BILASTIN-ZUSAMMENSETZUNG ZUR EINMAL TÄGLICHEN PARENTERALEN VERABREICHUNG
COMPOSITION DE BILASTINE POUR UNE ADMINISTRATION PARENTÉRALE UNIQUOTIDIENNE

(30) Priority: 17.02.2022 EP 22382134
(43) Date of publication of application: 25.12.2024
(73) Proprietor: FAES FARMA, S.A., 48940 Leioa - Vizcaya (ES)
(72) Inventor: HERNÁNDEZ HERRERO, Gonzalo, 48940 Leioa - Vizcaya (ES); EGUSQUIAGUIRRE MARTÍN, Susana Patricia, 48940 Leioa - Vizcaya (ES); FERNÁNDEZ HERNANDO, María Nieves, 48940 Leioa - Vizcaya (ES); ARRANZ GUTIÉRREZ, Paula, 48940 Leioa - Vizcaya (ES); GONZALO GOROSTIZA, Ana, 48940 Leioa - Vizcaya (ES); ARANA REY, Eider, 48940 Leioa - Vizcaya (ES); OTERO ESPINAR, Francisco Javier, 15782 Santiago de Compostela - A Coruña (ES); DÍAZ TOMÉ, Victoria, 15782 Santiago de Compostela - A Coruña (ES)
(74) Representative: ABG Intellectual Property Law, S.L.
(86) International application number: PCT/EP2023/053959
(87) International publication number: WO 2023/156559

(56) References cited:
- EP-A1- 3 725 298
- WO-A1-2019/141563

## Description

### FIELD OF THE INVENTION

The present invention relates to aqueous pharmaceutical compositions comprising bilastine and a β-cyclodextrin suitable for once-daily parenteral administration and their use as antihistamine and antiallergic pharmaceutical compositions.

### BACKGROUND

Antihistamines are compounds that are generally used in the symptomatic treatment of different allergic diseases or disorders since many of their symptoms (itchy eyes, runny nose, itchy skin, etc.) are caused by the action of histamine. Indeed they have been known for a long time - some of them even in the mid-twentieth century - for the treatment of numerous diseases and processes, such as allergic and non-allergic rhinitis and conjunctivitis; acute and chronic urticaria; control of itching and scratching of various dermatological (e.g. bites, dermatitis) and non-dermatological (e.g., metabolic itching) causes; symptomatic treatment of catarrhal respiratory infections and nonspecific cough; motion sickness, nausea and dizziness, and for the minor treatment of insomnia and anorexia (lack of appetite).

Antihistamines can be typically administered orally (as tablets, syrups, or drops) or topically (cream, eye drops, nasal spray, or ear drops).

Oral antihistamines can offer relief from several symptoms of allergy, however they have a delayed onset of action when compared with topical ocular agents.

The topical route is suitable for eyes, nose, or cutaneous disease (eye drops, nasal spray, cream, gel). If the allergic symptoms are primarily ocular, then topical treatments appear to be the treatments of choice as they have faster onset of action (within minutes) than systemic agents, and, thus, are readily able to retard the allergic response. Topical antihistaminic agents also show fewer adverse effects because of the lower doses required to penetrate the conjunctivae and the negligible serum levels from topical use. However, the use of topical antihistaminic agents is quite limited to ocular allergic reactions, therefore such topical formulations cannot be applied to a larger spectrum of diseases with their corresponding symptoms.

The parenteral route is usually employed for the prevention or treatment of serious and acute episodes of allergy. This route is only possible with some first-generation antihistamines, which are known for causing sedation and cognitive and psychomotor impairment. There is currently a lack of effective and well-tolerated options for parenteral treatment, especially formulations suitable for short-term treatment as single therapy, or for severe cases of histamine-mediated hypersensitivity reactions, when immediate action is required or when parenteral formulation is preferred.

Documents EP 0818454 A1 and EP 0580541 A1 disclose benzimidazole compounds with selective H₁ antihistaminic activity and devoid of arrhythmogenic effects. Patent application EP 3040334 A1 also discloses benzimidazole compounds having potent selective H₁ antihistaminic activity, lacking activity on the central nervous system and on the cardiovascular system.

A particular compound with the above properties is 2-[4-(2-{4-[1-(2-Ethoxyethyl)-1H-benzimidazol-2-yl]-1-piperidinyl}ethyl)phenyl]-2-methylpropanoic acid, also known as bilastine, having formula: 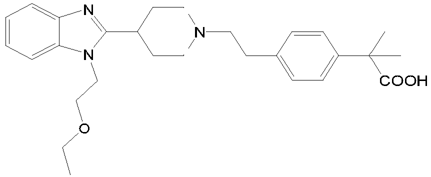 and developed by Faes Farma, Spain. Bilastine is a second-generation H₁ antagonist benzimidazole compound with no sedative side effects, no cardiotoxic effects, and no hepatic metabolism. In addition, bilastine has proved to be effective for the symptomatic treatment of allergic rhinoconjunctivitis and urticaria.

WO 2019/141563 discloses ophthalmic pharmaceutical compositions containing bilastine. These compositions require the presence of a gelling agent in order for the composition to provide a prolonged action in the eye and, therefore, would not be suitable for parenteral administration.

In case of allergy emergency, namely a severe event of histamine-mediated type I hypersensitivity reaction, immediate action is required as topical formulations may not be sufficiently effective while oral antihistaminic agents may not act within a reasonable timeframe. Parenteral administration of antihistamines may provide a solution to the lack of agents for allergy emergency, in particular, intravenous and intramuscular formulations seem well suited for a rapid and effective treatment of acute episodes of allergy. To date, only three injectable antihistaminic agents are known: Dexchlorpheniramine (trade name Polaramine^{®}), cetirizine hydrochloride (trade name Quzyttir^{®}) and diphenhydramine (trade name Benadryl^{®} - withdrawn from the US market - and several generic formulations).

Dexchlorpheniramine is a standard antihistaminic agent for allergy emergency events. It is the only injectable antihistaminic regulated in Europe, however, like other first generation H₁-antihistamine agents, may induce extreme sleepiness, dizziness or drowsiness. In some cases, up to four doses of dexchlorpheniramine per day may be necessary.

Cetirizine is a second generation antihistaminic compound that has been approved by FDA for intravenous administration in October 2019.

Diphenhydramine is a first generation H₁-antihistamine and has been approved by FDA as injectable formulation in a concentration of 50 mg of diphenhydramine hydrochloride per mL, however, like other first generation H₁-antihistamine agents, may induce extreme sleepiness, dizziness or drowsiness. In some cases, up to eight doses of dexchlorpheniramine per day may be necessary.

Other disclosures in the art which address the treatment of emergency allergic symptoms by administering parenteral antihistaminic formulations are quite rare.

EP1005865B1 discloses a process for making an antihistaminic and antiallergic composition that comprises 1) making a fine powdered blend of Nimesulide and Cetrizine and 2) dissolving the uniform blend in solvent suitable for parenteral administration at a temperature between 25 and 35 °C.

R. Corcóstegui et al. (Drugs R D 2006, 7(4), 219-231) compares the *in vivo* antihistaminic and antiallergic properties of bilastine, cetirizine and fexofenadine via different administrations routes (oral and intravenous) in various animal models. Bilastine was administered to rats intravenously in a tartaric acid solution five minutes before inducing papules by intradermal injection of histamine and the resulting increase of the capillary permeability was determined as a measure of the intensity of the histamine-mediated allergic reaction. Due to the low water solubility of bilastine (about 0.5 mg/ml at pH 5-8), a large volume of the bilastine composition disclosed in this document would be required to administer a therapeutically effective dose, which would prevent its efficient parenteral administration and the development of a pharmaceutically acceptable parenteral formulation. Of course, such a composition would not be suitable for a once-daily administration.

A parenteral composition with an optimal pH containing a high concentration of bilastine suitable for once-daily treatment/prevention as single therapy or for treating/preventing severe cases of histamine-mediated type I hypersensitivity reactions (such as acute urticaria, chronic urticaria, acute exacerbation of chronic urticaria, immediate-type-hypersensitivity reactions, anaphylaxis or angioedema) is not known in the art.

### BRIEF DESCRIPTION OF THE INVENTION

The inventors of the present invention have surprisingly found that the parenteral formulations of the invention result in a fast drug onset of action, very high efficacy and without side effects (the formulations are virtually as safe as the placebo and do not generate sleepiness nor drowsiness in contrast to known marketed formulations).

These compositions allow a very high concentration of bilastine and at a pH suitable for parenteral administration.

Unexpectedly, the formulation of the invention was found to be more efficient than known marketed formulations. In particular, the formulation of the invention showed better efficacy results than intramuscular administration of Polaramine^{®}, a strong antihistaminic agent for allergy emergency events that may induce extreme sleepiness, dizziness or drowsiness. Notably, the formulation of the invention does not induce sleepiness, dizziness nor drowsiness.

Also unexpectedly, when administered via intravenous or intramuscular routes, the formulation of the invention results at least in one of improved drug onset of action, improved efficacy, or less side effects, when compared to other bilastine compositions, when compared to other routes of administration, or when compared to other antihistamine compositions administrated via similar routes of administration.

Additionally, the composition of the invention has been found to be effective within 15 minutes after administration and maintained for 24 hours, which makes it particularly useful when immediate action is required, for example in the prevention/treatment of acute type I hypersensitivity reactions either alone or as an adjunctive therapy.

Further, due to the maintained effectiveness for up to 24 hours, the composition of the invention can be used for once-daily administration as opposed to current injectable antihistaminic treatments.

Consequently, in a first aspect, the invention provides an aqueous parenteral pharmaceutical composition comprising:
a) between 0.96% w/v and 2.60% w/v of bilastine, or a pharmaceutically acceptable salt or solvate thereof, and
b) between 10% w/v and 30% w/v of a β-cyclodextrin selected from unmodified β-cyclodextrin, C₁-C₆ alkyl-β-cyclodextrin, C₁-C₆ hydroxyalkyl β-cyclodextrin, C₁-C₆ carboxyalkyl-β-cyclodextrin, carbonyl-β-cyclodextrin, C₁-C₆ sulfoalkylether β-cyclodextrin and mixtures thereof, and
wherein the pH value of the composition is between 3.0 and 7.2, both lower and upper limits of the range included, and wherein parenteral is selected from intravenous or intramuscular.

Another aspect of the invention refers to the aqueous parenteral pharmaceutical composition of the invention for use as a medicament.

In a further aspect, the invention is directed to the aqueous parenteral pharmaceutical composition of the invention for use in the treatment and/or prevention of an allergic disease or disorder or allergic symptoms.

Another aspect of this invention refers to the use of the aqueous parenteral pharmaceutical composition of the invention in the manufacture of a medicament for the treatment and/or prevention of an allergic disease or disorder or allergic symptoms.

Another aspect of the present invention refers to a method for the treatment and/or prevention of an allergic disease or disorder or allergic symptoms, the method comprising administering to a subject in need of such a treatment and/or prevention a therapeutically effective amount of the aqueous parenteral pharmaceutical composition of the invention.

These aspects and preferred embodiments thereof are also additionally described further down in the description and defined in the claims.

### BRIEF DESCRIPTION OF THE FIGURES

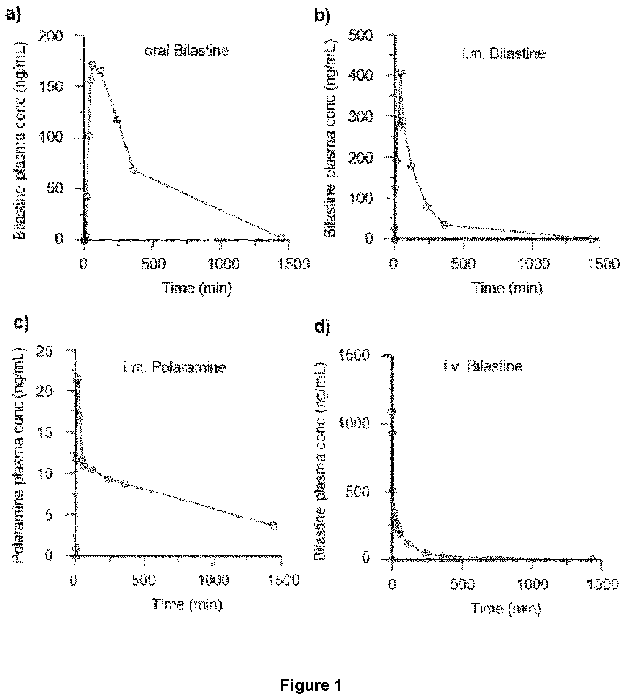

Figure 1 shows the mean plasma concentration/time curves (0 - 24h) for: a) oral Bilastine (Bilaxten^{®}), b) intramuscular (i.m.) Bilastine, c) intramuscular (i.m.) Polaramine^{®} and d) intravenous (i.v.) Bilastine.

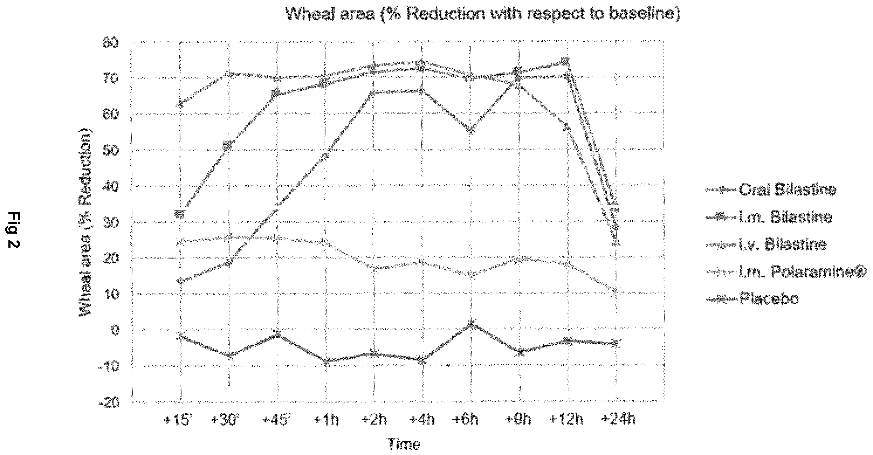

Figure 2 is a graph showing the comparison of i.m. Bilastine, i.v. Bilastine, i.m. Polaramine^{®}, oral Bilastine and Placebo in the % reduction of the wheal area caused by histamine injection with respect to the baseline. Curve *i.m. Bilastine:* P ≤ 0.001 vs i.m. Polaramine^{®} (time data points ≥ 30') and ns at 15' (ns: not significant, P > 0.05); P ≤ 0.001 vs i.v. Bilastine (15', 30' and 12h), P > 0.05 for the rest of the data points; P ≤ 0.001 vs placebo (all time data). Curve *i.v. Bilastine:* P ≤ 0.001 vs i.m. Polaramine^{®} (15'-12h), P ≤ 0.05 (24h); P ≤ 0.001 (15'-12h), P = 0.002 (24h) vs placebo. Curve oral Bilastine: P ≤ 0.001 (30', 45'), P ≤ 0.05 (15', 1h), ns (remainder) vs i.m. Bilastine; P ≤ 0.001 (15'-1h), P ≤ 0.05 (12h), ns (remainder) vs i.v. Bilastine; P ≤ 0.001 (1h-12h), P ≤ 0.05 (24h), ns (remainder) vs i.m. Polaramine^{®}; P ≤ 0.001 (45'-24h), P ≤ 0.05 (15', 30') vs placebo. Curve i.m. Polaramine^{®}: P ≤ 0.001 (15'-4h, 9h), P ≤ 0.05 (12h, 24h), ns (6h) vs placebo.

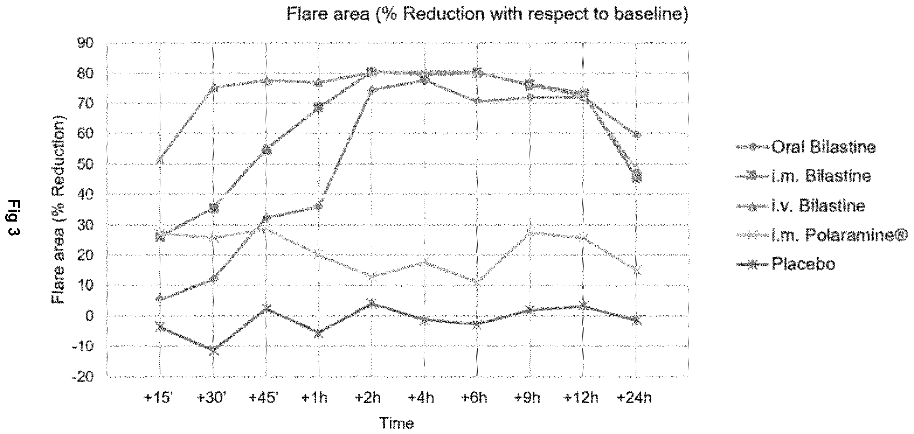

Figure 3 is a graph showing the comparison of i.m. Bilastine, i.v. Bilastine, i.m. Polaramine^{®}, oral Bilastine and Placebo in the % reduction of the flare area caused by histamine injection with respect to the baseline. Curve *i.m. Bilastine:* P ≤ 0.001 vs i.m. Polaramine^{®} (45'-24h) and ns at 15' and 30'; P ≤ 0.001 (15', 30' and 45'), P ≤ 0.05 (1h), ns for the remainder of the data points vs i.v. Bilastine; P ≤ 0.001 vs placebo (all time data). Curve *i.v. Bilastine:* P ≤ 0.001 vs i.m. Polaramine^{®} (15'-24h); P ≤ 0.001 (15'-24h) vs placebo. Curve oral Bilastine: P ≤ 0.001 (45', 1h), P ≤ 0.05 (15', 30', 24h), ns (remainder) vs i.m. Bilastine; P ≤ 0.001 (15'-1h), P ≤ 0.05 (2h, 24h), ns (remainder) vs i.v. Bilastine; P ≤ 0.001 (2h-24h), P ≤ 0.05 (15', 30', 1h), ns (45') vs i.m. Polaramine^{®}; P ≤ 0.001 (45'-24h), P ≤ 0.05 (30'), ns (15') vs placebo. Curve i.m. Polaramine^{®}: P ≤ 0.001 (30', 45'), P ≤ 0.05 (15', 1h, 4h - 24h), ns (2h) vs placebo.

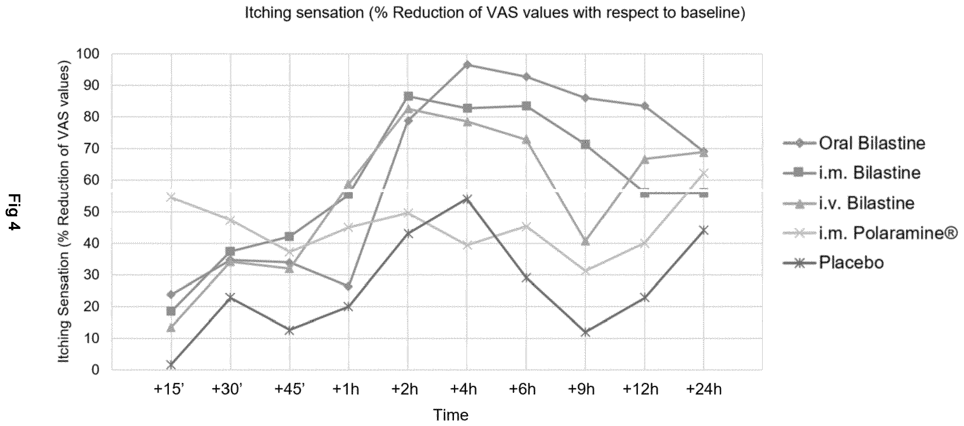

Figure 4 is a graph showing the comparison of i.m. Bilastine, i.v. Bilastine, i.m. Polaramine^{®}, oral Bilastine and Placebo in the % reduction of the VAS (Visual Analogue Scale) values related to the itching sensation caused by histamine injection with respect to the baseline. Curve *i.m. Bilastine:* P ≤ 0.001 (2h-9h) and ns (remainder) vs i.m. Polaramine^{®}; ns vs i.v. Bilastine for all time data points; P ≤ 0.001 (2h, 4h), P ≤ 0.05 (1h, 6h, 9h), ns (remainder) vs placebo. Curve *i.v. Bilastine:* P ≤ 0.05 (2h, 4h, 6h), ns (remainder) vs i.m. Polaramine^{®}; P ≤ 0.001 (2h), P ≤ 0.05 (1h, 4h), ns (remainder) vs placebo. Curve oral Bilastine: ns vs i.m. Bilastine (all data points); ns vs i.v. Bilastine (all data points); P ≤ 0.001 (9h), P ≤ 0.05 (2h, 4h, 6h, 12h), ns (remainder) vs i.m. Polaramine^{®}; P ≤ 0.05 (2h-24h), ns (15'-1h) vs placebo. Curve i.m. Polaramine^{®}: P ≤ 0.05 (15'), ns (remainder) vs placebo.

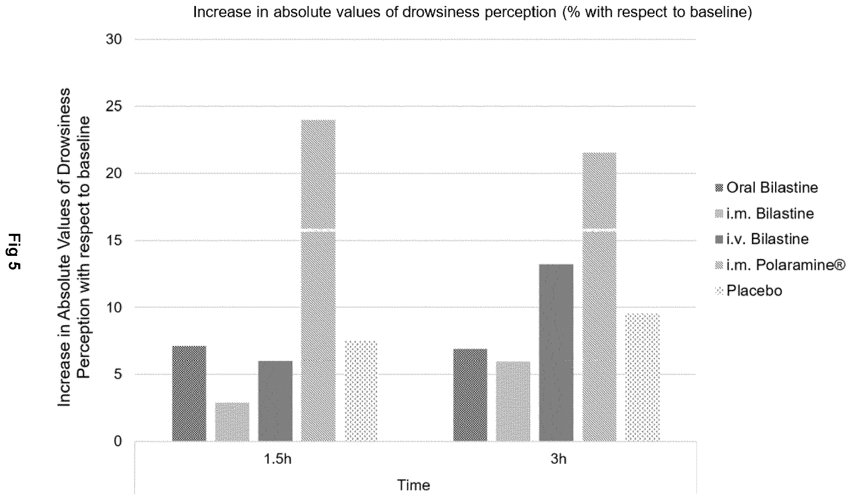

Figure 5 shows the increase in absolute values of drowsiness perception with respect to baseline when using, i.m. Bilastine, i.v. Bilastine, i.m. Polaramine^{®}, oral Bilastine and Placebo. Curve *i.m. Bilastine:* P ≤ 0.05 vs i.m. Polaramine^{®}; ns vs i.v. Bilastine and placebo. Curve *i.v. Bilastine:* ns vs i.m. Polaramine^{®}; ns vs placebo. Curve oral Bilastine: ns vs i.m. Bilastine, i.v. Bilastine and placebo (3h and 1.5h); P ≤ 0.05 vs i.m. Polaramine^{®} (1.5h and 3h). Curve i.m. Polaramine^{®}: P ≤ 0.05 (1.5h), ns (3h) vs placebo.

### DETAILED DESCRIPTION OF THE INVENTION

The term "alkyl" refers to a linear or branched alkane derivative containing from 1 to 6 ("C₁-C₆ alkyl"), preferably from 1 to 3 ("C₁-C₃ alkyl"), carbon atoms and which is bound to the rest of the molecule through a single bond. Illustrative examples of alkyl groups include methyl, ethyl, n-propyl, i-propyl, n-butyl, i-butyl, t-butyl, pentyl, hexyl.

The expression "pharmaceutically acceptable" refers to compositions and molecular entities that are physiologically tolerable and do not typically produce an allergic reaction or a similar unfavourable reaction as gastric disorders, dizziness and suchlike, when administered to a human or animal. Preferably, the term "pharmaceutically acceptable" means it is approved by a regulatory agency of a state or federal government or is included in the U.S. Pharmacopoeia or other generally recognized pharmacopoeia for use in animals, and more particularly in humans.

In the present application, all the disclosed percentages refer to w/v percentage unless otherwise stated.

When a range is indicated in the present document, both lower and upper limits are included in said range.

All the embodiments disclosed in relation to an aspect of the invention are applicable to the other aspects.

The inventors have surprisingly found that the combination of bilastine with at least a β-cyclodextrin is optimal for parenteral administration, showing a fast onset of action, virtually no side-effects (especially in terms of drowsiness which characterizes other injectable antihistaminic formulations currently approved by regulatory agencies) and an excellent prolonged action at a systemic level which allows for the first time the provision of a once-daily parenteral formulation comprising bilastine. Thus, when administered via intravenous or intramuscular routes, the formulation of the invention results at least in one of improved drug onset of action, improved efficacy, or less side effects, when compared to other bilastine compositions, when compared to other routes of administration, or when compared to other antihistamine compositions administrated via similar routes of administration.

Consequently, in a first aspect, the invention provides an aqueous parenteral pharmaceutical composition comprising:
a) between 0.96% w/v and 2.60% w/v of bilastine, or a pharmaceutically acceptable salt or solvate thereof, and
b) between 10% w/v and 30% w/v of a β-cyclodextrin selected from unmodified β-cyclodextrin, C₁-C₆ alkyl-β-cyclodextrin, C₁-C₆ hydroxyalkyl β-cyclodextrin, C₁-C₆ carboxyalkyl-β-cyclodextrin, carbonyl-β-cyclodextrin, C₁-C₆ sulfoalkylether β-cyclodextrin and mixtures thereof, and
wherein the pH value of the composition is between 3.0 and 7.2, both lower and upper limits of the range included, and wherein parenteral is selected from intravenous or intramuscular.

### Bilastine

The aqueous parenteral composition of the invention comprises bilastine, of formula, 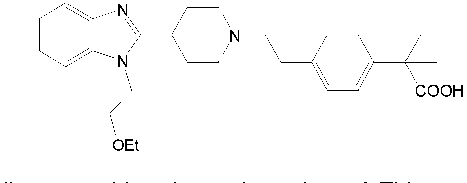 or a pharmaceutically acceptable salt or solvate thereof. This compound is 2-[4-(2-{4-[1-(2-ethoxyethyl)-1H-benzimidazol-2-yl]-1-piperidinyl}ethyl)phenyl]-2-methylpropanoic acid, also known as bilastine. The synthesis of bilastine has been described, for example, in documents EP 0818454 A1, EP 0580541 A1 and EP 3040334 A1.

Bilastine may be in the form of a salt or solvate, preferably a pharmaceutically acceptable salt or solvate.

By way of illustration, said salts can be acid addition salts, base addition salts or metal salts, and can be synthesized from the parent compound by means of conventional chemical processes known by the persons skilled in the art. Such salts are generally prepared, for example, by reacting the free compound with a stoichiometric amount of the suitable base or acid in water or in an organic solvent or in a mixture of the two. Nonaqueous media such as ether, ethyl acetate, ethanol, acetone, isopropanol or acetonitrile are generally preferred. Illustrative examples of acid addition salts include inorganic acid addition salts such as, for example, hydrochloride, hydrobromide, hydroiodide, sulfate, nitrate, phosphate, etc., organic acid addition salts such as, for example, acetate, maleate, fumarate, citrate, oxalate, succinate, tartrate, malate, mandelate, methanesulfonate, p-toluenesulfonate, camphorsulfonate, etc. Illustrative examples of base addition salts include inorganic base salts such as, for example, ammonium salts and organic base salts such as, for example, ethylenediamine, ethanolamine, N,N-dialkylenethanolamine, triethanolamine, glutamine, amino acid basic salts, etc. Illustrative examples of metal salts include, for example, sodium, potassium, calcium, magnesium, aluminium and lithium salts.

The term "solvate" according to this invention is to be understood as meaning any form of the active compound which has another molecule (most likely a polar solvent) attached to it via non-covalent bonding. Examples of solvates include hydrates and alcoholates. Solvation methods are generally known in the state of the art.

The compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or a nitrogen by ¹⁵N-enriched nitrogen are within the scope of this invention.

In an embodiment, bilastine, or a pharmaceutically acceptable salt or solvate thereof, is present in the pharmaceutical composition of the invention at a concentration between 0.96% and 2.60% w/v. A concentration of between 0.96% and 2.60% w/v means that there are between 9.6 mg and 26.0 mg of bilastine or a pharmaceutically acceptable salt or solvate thereof, in 1 mL of aqueous composition. In an embodiment, bilastine, or a pharmaceutically acceptable salt or solvate thereof, is present in the pharmaceutical composition of the invention at a concentration between 1% and 2.6% w/v. In an embodiment, bilastine, or a pharmaceutically acceptable salt or solvate thereof, is present in the pharmaceutical composition of the invention at a concentration between 1.00% and 2.60% w/v.

In an embodiment, bilastine, or a pharmaceutically acceptable salt or solvate thereof, is present in the pharmaceutical composition of the invention at a concentration between 0.96% and 1.5% w/v. In an embodiment, bilastine, or a pharmaceutically acceptable salt or solvate thereof, is present in the pharmaceutical composition of the invention at a concentration between 0.96% and 1.50% w/v. In another embodiment, bilastine, or a pharmaceutically acceptable salt or solvate thereof, is present in the pharmaceutical composition of the invention at a concentration between 1.0% and 1.4% w/v. In another embodiment, bilastine, or a pharmaceutically acceptable salt or solvate thereof, is present in the pharmaceutical composition of the invention at a concentration between 1.00% and 1.40% w/v. In an embodiment, the composition comprises between 1.05% w/v and 1.35% w/v of bilastine or a pharmaceutically acceptable salt or solvate thereof. In a further embodiment, it comprises between 1.1% w/v and 1.3% w/v of bilastine or a pharmaceutically acceptable salt or solvate thereof, such as for example between 1.14% w/v and 1.26% w/v, or even 1.2% w/v. In a further embodiment, it comprises between 1.10% w/v and 1.30% w/v of bilastine or a pharmaceutically acceptable salt or solvate thereof, such as for example between 1.14% w/v and 1.26% w/v, or even 1.20% w/v.

In the present invention, unless it is stated otherwise or not feasible, a value is to be considered as rounded up to the last indicated decimal. For example, a number such as "1.10" comprises the range 1.095 to 1.104. Likewise, a value such as "1.5" comprises the range 1.45 to 1.54 or a value such as "1.0" comprises the range 0.95 to 1.04.

The mass of bilastine can be calculated from the % w/v values accordingly. In a particular embodiment, the pharmaceutical composition is a unit dose. In an embodiment, the pharmaceutical composition is a unit dose comprising from 9.6 mg to 28.0 mg, or even from 9.6 mg to 22.0 mg, of bilastine. In another embodiment, the pharmaceutical composition is a unit dose comprising from 10 mg to 18 mg of bilastine, or from 11 mg to 15 mg of bilastine.

In an embodiment, the volume of the unit dose of the composition of the invention is from 0.5 to 1.5 mL. In another embodiment, the volume of the unit dose of the composition of the invention is 0.8-1.5 mL or even 0.8-1.2 mL.

The term "unit dose" refers to the amount administered to a subject or patient in a single dose.

It has been found that the β-cyclodextrin increases the solubility of Bilastine, so that a composition with a high concentration of Bilastine can be obtained. Bilastine, or the pharmaceutically acceptable salt or solvate thereof, is completely dissolved in the pharmaceutical composition of the invention, i.e, without any significant signs of precipitation.

Bilastine concentration can be determined, for example, by UV spectrophotometry at 254 nm.

In an embodiment, bilastine or a salt or solvate thereof is the only active pharmaceutical ingredient in the composition of the invention.

### Cyclodextrins

A cyclodextrin (CD) is a cyclic structure composed of 5 or more β-D-glucopyranose units linked at the 1,4 positions, typically having 6 (α-cyclodextrin), 7 (β-cyclodextrin), 8 (γ-cyclodextrin) or 9 (δ-cyclodextrin) sugar units in one cyclodextrin molecule.

The MS value (average molar degree of substitution) is the average number of moles of substituent groups per glucopyranose mol. For example, in the case of β-cyclodextrins, the degree of substitution/modification (DS) is the average number of substituents per β-cyclodextrin core and can be calculated by multiplying the MS value by 7 (the β-cyclodextrin comprises 7 sugar units per cyclodextrin molecule).

Both amorphous and crystalline cyclodextrins are within the scope of the present application. As used herein the term "cyclodextrin" may refer to a cyclodextrin or a cyclodextrin derivative. Cyclodextrins are commercially available or may be synthesized by methods well-known in the art. Examples of cyclodextrins include, but are not limited to, modified or unmodified α-, β-, γ- and δ-cyclodextrins.

The cyclodextrins of the present invention are β-cyclodextrins. Derivatives of cyclodextrins and particularly of the β-cyclodextrins of the invention include those wherein some or all of the OH groups are converted to OR groups. Said derivatives include those with C₁₋₆ alkyl groups such as e.g. methylated, ethylated, propylated and butylated cyclodextrins, wherein R is a methyl, ethyl, propyl or butyl group; and those with C₁-C₆ hydroxyalkyl substituted groups such as e.g. hydroxypropyl cyclodextrins or hydroxyethyl cyclodextrins, wherein R is a -CH₂CH(OH)CH₃ or a CH₂CH₂OH group branched cyclodextrins such as maltose-bonded cyclodextrins; cationic cyclodextrins; quaternary ammonium; anionic cyclodextrins such as carboxymethyl cyclodextrins, cyclodextrin sulfates and cyclodextrin succinates; amphoteric cyclodextrins such as carboxymethyl/quaternary ammonium cyclodextrins. Other specific modifications include one or more hydroxyalkyl ether (e.g. R is C₁₋₆alkylenehydroxy) moieties; C₁-C₆ sulfoalkyl ether (e.g. R is C₂₋₆ alkyleneSO₃⁻) moieties; C₁-C₆ carboxyalkyl (e.g. R is C(O)C₁₋₆alkyl) moieties; substituted phenoxy moieties; tryptophan moieties; or mixtures thereof. The total number of OR groups per cyclodextrin molecule is defined as the degree of substitution/modification.

In the present invention, the β-cyclodextrin is selected from the group consisting of unmodified β-cyclodextrin, C₁-C₆ alkyl-β-cyclodextrin, C₁-C₆ hydroxyalkyl β-cyclodextrin, C₁-C₆ carboxyalkyl-β-cyclodextrin, carbonyl-β-cyclodextrin, C₁-C₆ sulfoalkylether β-cyclodextrin and mixtures thereof.

In one embodiment the β-cyclodextrin is a C₁-C₆ alkyl-β-cyclodextrin. Preferred alkyl-β-cyclodextrins include methyl-β-cyclodextrin; dimethyl-β-cyclodextrin; trimethyl-β-cyclodextrin; ethyl-β-cyclodextrin; diethyl-β-cyclodextrin; propyl-β-cyclodextrin; and butyl-β-cyclodextrin. In a more preferred embodiment the β-cyclodextrin is selected from the group consisting of methyl-β-cyclodextrin or dimethyl-β-cyclodextrin. In the context of the present invention, when the term "alkyl-β-cyclodextrin" is used, it is meant to include β-cyclodextrins wherein the alkyl moiety is optionally substituted but excluding hydroxyalkyl-β-cyclodextrins.

The C₁-C₆ alkyl β-cyclodextrin preferably has a degree of substitution/modification of from 1 to 18, from 3 to 16, from 4 to 14, from 4 to 12.6, and more preferably from 4 to 6.

In another embodiment the β-cyclodextrin is a C₁-C₆ hydroxyalkyl-β-cyclodextrin. Preferred hydroxyalkyl-β-cyclodextrins include hydroxyethyl-β-cyclodextrin; hydroxypropyl-β-cyclodextrin (which is equivalent to 2-hydroxypropyl-β-cyclodextrin) and 2-hydroxybutyl-β-cyclodextrin. In a more preferred embodiment the β-cyclodextrin is hydroxypropyl-β-cyclodextrin (2-hydroxypropyl-β-cyclodextrin; HPBCD or HP-β-CD).

The C₁-C₆ hydroxyalkyl β-cyclodextrin, and particularly the hydroxypropyl-β-cyclodextrin, preferably has a degree of substitution/modification of from 1 to 14, more preferably from 2 to 8. In a further embodiment, it has a degree of substitution from 3 to 7, preferably from 3 to 6.

In an embodiment, the hydroxyalkyl β-cyclodextrin, and particularly the hydroxypropyl-β-cyclodextrin, preferably has an average molar degree of substitution of from 0.42 to 0.86, or from 0.50 to 0.75.

In a further embodiment the β-cyclodextrin is a C₁-C₆ carboxyalkyl-β-cyclodextrin. Preferred C₁-C₆ carboxyalkyl-β-cyclodextrins for use herein include carboxymethyl-β-cyclodextrin and (2-carboxyethyl)-β-cyclodextrin.

In a further embodiment the β-cyclodextrin is a C₁-C₆ sulfoalkylether β-cyclodextrin. A preferred sulfoalkylether-β-cyclodextrin for use herein is sulfobutylether-β-cyclodextrin.

The sulfoalkylether-β-cyclodextrin preferably has a degree of substitution/modification from 1 to 14, preferably from 1 to 7.

In an embodiment the β-cyclodextrin is selected from the group consisting of C₁-C₆ alkyl-β-cyclodextrin, C₁-C₆ hydroxyalkyl β-cyclodextrin, C₁-C₆ carboxyalkyl-β-cyclodextrin, C₁-C₆ sulfoalkylether β-cyclodextrin and mixtures thereof.

In a preferred embodiment the β-cyclodextrin is a pharmaceutically acceptable β-cyclodextrin.

In a particular embodiment, the β-cyclodextrin is present in the composition in an amount from 10% to 30% w/v. Here, w/v means weight/volume percentage concentration (g/100 mL), e.g. when the β-cyclodextrin is present in the composition in an amount from 10% to 30% w/v it means that there are between 100 mg and 300 mg of β-cyclodextrin per 1 mL of aqueous composition.

In specific embodiments, the β-cyclodextrin is present in an amount between 10% and 25% w/v. In another embodiment, the β-cyclodextrin is present in an amount between 12% w/v and 22% w/v, or between 15% w/v and 20% w/v. In a further embodiment, the β-cyclodextrin is present in an amount between 16% and 18% w/v, such as for example 17% w/v.

In a further embodiment, the pharmaceutical composition is a unit dose comprising 100-330 mg of the β-cyclodextrin. In another embodiment, the pharmaceutical composition is a unit dose comprising 100-200 mg of the β-cyclodextrin, or even 150-200 mg of the β-cyclodextrin.

In a further embodiment, the pharmaceutical composition is a unit dose comprising 9.6-28 mg of bilastine and 100-330 mg of the β-cyclodextrin. In another embodiment, the pharmaceutical composition is a unit dose comprising 10-18 mg of bilastine, or 11-15 mg of bilastine, and 100-200 mg of the β-cyclodextrin, or even 150-200 mg of the β-cyclodextrin.

### pH

The aqueous pharmaceutical composition of the invention has been developed for parenteral uses and/or administration, i.e. the aqueous parenteral pharmaceutical composition is adapted to these purposes. The pH is an important aspect of parenteral preparations which should have a pH close to the physiological one. However, a compromise can be found between the pH ensuring stability of the drug substance and the physiological one, as the pH could have an influence on the stability of the preparation. The acceptable range is pH 3 - 9 for intravenous and intramuscular injection due to potential irritation issue. An unsuitable pH could promote local irritation and an increased pain during the administration.

In an embodiment, the pharmaceutical composition has a pH value comprised between 3.0 and 7.2, both lower and upper limits included. In an embodiment, the pharmaceutical composition has a pH value comprised between 4.0 and 7.2, both lower and upper limits included. In another embodiment, the pharmaceutical composition has a pH value comprised between 4.0 and 7.0, both lower and upper limits included. In a further embodiment, the composition of the invention has a pH value between 4.0 and 6.0, both lower and upper limits included, such as for example between 4.5 and 5.5. In some embodiments the pH of the composition of the invention is 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1 or 7.2.

In an embodiment, the pH of the composition is 5. In the context of pH values, it must be readily understood that a pH value of 5 may also be identified as corresponding to a pH range of from 4.5 to 5.4.

The composition can comprise a pH-adjusting agent to obtain the desired pH value of the composition.

In an embodiment, the composition comprises a pH-adjusting agent selected from the group consisting of hydrochloric acid, boric acid, acetic acid, sodium hydroxide, potassium hydroxide, or a combination thereof. Preferably, the pH-adjusting agent is selected from hydrochloric acid, boric acid, acetic acid, and mixtures therefor; more preferably it is hydrochloric acid, such as concentrated hydrochloric acid (37% w/w).

pH can be determined by measuring the pH directly in the composition using conventional methods, for example using a pH electrode, preferably at 23°C.

### Further embodiments

The composition of the invention further comprises balance water. That is, it comprises water to reach the desired final volume of the composition.

The water in the composition of the invention is preferably water for injection, such as distilled and/or sterile water for injection.

The term "water for injection" refers to water that is purified such that is suitable for parenteral administration; such as water that meets the USP (or foreign equivalent) requirements for water for injection.

The composition of the invention may comprise further pharmaceutically acceptable parenteral carriers or excipients. Suitable pharmaceutically acceptable parenteral excipients or carriers include, by way of non-limiting examples, preservatives, including antimicrobial preservatives and chemical preservatives, tonicity agents, buffering agents, antioxidants, chelating agents, bulking agents, solubilizing agents, surfactants, co-solvents and combinations thereof. Suitable pharmaceutical carriers or excipients are described, for example, in "Remington's Pharmaceutical Sciences" by E.W. Martin, 21st Edition, 2005.

Preservatives may include benzalkonium chloride, benthezonium chloride, benzyl alcohol, benzoic acid, chlorobutanol, m-cresol, methylparaben, propylparaben, butylparaben, phenol, 2-phenoxyethanol, phenyl mercuric nitrate, phenyl mercuric acetate, phenyl mercuric borate, thimerosal and mixtures thereof.

Tonicity agents typically used are sodium chloride, dextrose, glycerol, glycerine, mannitol, potassium chloride, propylene glycol and mixtures thereof.

Buffering agents may include acid/sodium/disodium/trisodium citrate, acid/sodium/potassium phosphate, sodium/acetic acid/glacial acetic acid/ammonium acetate, sodium hydroxide, tris acetate, ammonium sulfate, ammonium hydroxide, arginine, benzene sulfonic acid, benzoate sodium/acid, sodium bicarbonate, boric acid/sodium, sodium carbonate, carbon dioxide, diethanolamine, glucono delta lactone, glycine/glycine HCl, histidine/histidine HCl, hydrochloric acid, hydrobromic acid, lysine, maleic acid, meglumine, methanesulfonic acid, monoethanolamine, succinate sodium/disodium, sulfuric acid, tartrate sodium/acid, tromethamine and mixtures thereof.

Antioxidants may include ascorbic acid, acetylcysteine, ascorbyl palmitate, sodium ascorbate, butylated hydroxyl toluene, butylated hydroxyl anisole, citric acid, monothioglyercol, sulfurous acid salts (bisulfite, metabisulfite), methionine, sodium metabisulfite, potassium metabisulfite, acetone metabisulfite, cysteine hydrochloride, sodium dithionite, gentisic acid, sodium glutamate, glutathione, thioglycerol, propyl gallate, sodium sulfite, sodium bisulfite, sodium formaldehyde sulfoxylate, thiourea, alpha tocopherol and mixtures thereof.

Chelating agents may include disodium edetate, sodium edetate, edetate calcium disodium, DTPA, citric acid monohydrate, calcium versetamide, calteridol, ethylene diamine tetra acetic acid and mixtures thereof.

Bulking agents may include mannitol, sucrose, lactose, dextran, trehalose, sorbitol, glucose, raffinose, glycine, histidine, and mixtures thereof.

Solubilizing agents, surfactants and co-solvents may include surfactants such as polyoxyethylene sorbitan monooleate (Tween 80), sorbitan monooleate polyoxyethylene sorbitan monolaurate (Tween 20), lecithin, polyoxyethylene-polyoxypropylene copolymers (Pluronics), and alcohols such as ethanol, benzyl benzoate, dimethylacetamide, glycerin, sorbitol, polyethylene glycol, pyrrolidone, propylene glycol, castor oil, soybean oil, cottonseed oil, safflower oil, sesame oil, peanut oil, poppyseed oil, vegetable oil, and mixtures thereof.

In an embodiment, the pharmaceutical composition of the invention may comprise further pharmaceutically acceptable parenteral carriers or excipients in an amount of from 0 to 50% w/v, from 0 to 40% w/v or even from 0 to 30% w/v. That is, the composition may not include further pharmaceutically acceptable parenteral carriers or excipients (0% w/v) or may include further pharmaceutically acceptable parenteral carriers or excipients in a maximum amount of 50% w/v, 40% w/v or 30% w/v, respectively. In a particular embodiment, the pharmaceutical composition of the invention may comprise further pharmaceutically acceptable parenteral carriers or excipients in an amount of from 0 to 20% w/v or even from 0 to 10% w/v.

The expression "pharmaceutically acceptable excipient" refers to a vehicle, diluent, or adjuvant that is administered with the active ingredient.

In an embodiment, the composition of the invention does not comprise a gelling agent, such as hyaluronic acid, gellan gum, guar gum, locust bean gum, alginic acid, povidone, kappa-carrageenan, alginate gum, dextran, dextran sulfate, chitosan, or a salt thereof.

In a particular embodiment, the composition of the invention does not comprise hyaluronic acid or a salt thereof.

In an embodiment, the composition of the invention consists of:
a) between 0.96% w/v and 2.60% w/v of bilastine, or a pharmaceutically acceptable salt or solvate thereof,
b) between 10% w/v and 30% w/v of a β-cyclodextrin selected from unmodified β-cyclodextrin, C₁-C₆ alkyl-β-cyclodextrin, C₁-C₆ hydroxyalkyl β-cyclodextrin, C₁-C₆ carboxyalkyl-β-cyclodextrin, carbonyl-β-cyclodextrin, C₁-C₆ sulfoalkylether β-cyclodextrin and mixtures thereof,
c) a pH adjusting agent to provide a pH value between 3.0 and 7.2, both lower and upper limits of the range included,
d) water for injection, and
e) optionally, one or more pharmaceutically acceptable parenteral excipients or carriers selected from preservatives, including antimicrobial preservatives and chemical preservatives, tonicity agents, buffering agents, antioxidants, chelating agents, bulking agents, solubilizing agents, surfactants, co-solvents and mixtures thereof.

In an embodiment, the further pharmaceutically acceptable parenteral excipients or carriers are present in the composition in an amount from 0 to 50% w/v, or in an amount from 0 to 40% w/v, or in an amount from 0 to 30% w/v. In an embodiment, the further pharmaceutically acceptable parenteral excipients or carriers are present in the composition in an amount from 0 to 20% w/v, or in an amount from 0 to 10% w/v.

Preferably, the term one or more refers to from 1 to 5, or from 1 to 4. In an embodiment, it refers to 1, 2 or 3.

According to a particular embodiment, the composition of the invention consists of:
a) between 0.96% w/v and 2.60% w/v of bilastine, or a pharmaceutically acceptable salt or solvate thereof,
b) between 10% w/v and 30% w/v of a β-cyclodextrin selected from unmodified β-cyclodextrin, C₁-C₆ alkyl-β-cyclodextrin, C₁-C₆ hydroxyalkyl β-cyclodextrin, C₁-C₆ carboxyalkyl-β-cyclodextrin, carbonyl-β-cyclodextrin, C₁-C₆ sulfoalkylether β-cyclodextrin and mixtures thereof,
c) a pH adjusting agent to provide a pH value between 3.0 and 7.2, both lower and upper limits of the range included, and
d) water for injection.

In an embodiment, the pharmaceutical composition of the invention is a once-daily pharmaceutical composition.

The pharmaceutical composition of the invention is suitable for parenteral administration. In particular, it is suitable for intramuscular or intravenous; preferably, intramuscular administration.

The term "parenteral" refers to any method of administering a medicament to a patient that does not involve the digestive tract. The term "parenteral" comes from the Greek words "para" meaning beside or alongside, and "enteron" meaning intestine.

In the present invention, the term "parenteral" is selected from the group consisting of intramuscular administration and intravenous administration.

The present invention relates to an anti-histamine composition exhibiting a rapid onset of action. In this sense, an administration route such as intradermal will lead to a slow onset of action. In such a case, the inventors believe that the presence of excipients such as gelling agents will produce an even slower onset of action.

In a particular embodiment, the pharmaceutical composition of the invention is not suitable for subcutaneous administration or intradermal administration. In another particular embodiment, the pharmaceutical composition of the invention is not suitable for at least one of the following: subcutaneous administration, intrathecal administration, intra-arterial administration, intradermal administration, intraocular administration, or subconjunctival administration.

Both subcutaneous and intradermal administration routes are given by means of injections into the skin between the epidermal and dermal layers. Subcutaneous are generally administrated at an angle of 45 °, while intradermal are usually administrated at an angle of from 10° to 15 °. In both subcutaneous and intradermal administration routes, the volumes injected are lower than 0.5 mL, usually lower than 0.3 mL, preferably lower than 0.2 mL. Contrary to subcutaneous or intradermal administration routes, the volumes injected in intramuscular or intravenous administrations are usually equal to or greater than 0.3 mL, 0.5 mL, 0.75 mL or 1 mL.

In a particular embodiment, the pharmaceutical composition of the invention is a parenteral administration, wherein the injected volumes are comprised between 0.5 and 5 mL, between 0.5 and 4 mL, between 0.5 and 3 mL, between 0.5 and 2 mL, between 0.5 and 1.5 mL, preferably between 0.7 and 1.5 mL, more preferably between 0.8 and 1.2 mL, and even more preferably between 0.9 and 1.1 mL.

The present invention relates to parenteral administration, consisting of intramuscular or intravenous administration. Contrary to subcutaneous or intradermal administration routes, intramuscular administration is given at an angle greater than 45 °, preferably from 70 to 90 °, more preferably from 80 to 90°, most preferably at an angle of 90 °, relative to the skin. The skilled person will readily know the angle required to administrate a composition in an intramuscular fashion. Intravenous administration is usually given at a 25 ° angle.

The muscle sites commonly used for intramuscular administration are the gluteal, thigh, or shoulder muscles.

### Uses

Bilastine has been found to be an antagonist of histamine H₁ receptor and is thus useful in the treatment and/or prevention of diseases known to be susceptible to amelioration by antagonism of histamine H₁ receptor.

Therefore, an aspect of the invention refers to the aqueous parenteral pharmaceutical composition of the invention for use as a medicament.

Another aspect of the invention refers to the aqueous parenteral pharmaceutical composition of the invention for use in the treatment and/or prevention of a disease, disorder or condition susceptible to amelioration by antagonism of histamine H₁ receptor. Such diseases, disorders or conditions are, for example, allergic diseases, allergic disorders and symptoms derived from allergies.

In a preferred embodiment, the invention is directed to the aqueous parenteral pharmaceutical composition of the invention for use in the treatment and/or prevention of allergic diseases, allergic disorders, or allergic symptoms. Preferably, the allergic disease, allergic disorder, or allergic symptom is selected from rhinitis, conjunctivitis, rhinoconjunctivitis, hay fever, dermatitis, eczema, erythema, pruritus, itchiness, urticaria, chronic urticaria, asthma, anaphylaxis, acute urticaria, acute exacerbation of chronic urticaria, immediate-type-hypersensitivity reactions, angioedema, food allergy or drug allergy.

In an embodiment, the composition of the invention is used in the prevention and/or treatment of histamine-mediated type I hypersensitivity reactions, such as allergic rhinitis, conjunctivitis, rhinoconjunctivitis, asthma, dermatitis, urticaria, acute urticaria, chronic urticaria, anaphylaxis, angioedema, food allergy, drug allergy, hay fever, eczema, erythema, pruritus or itchiness. In a preferred embodiment, the composition is used in the prevention and/or treatment of acute urticaria, chronic urticaria, acute exacerbation of chronic urticaria, immediate-type-hypersensitivity reactions, anaphylaxis or angioedema.

In the present invention, the parenteral route (IM or IV) can be employed for the prevention or treatment of serious and acute episodes of allergy. In a preferred embodiment, the composition of the invention is used in the short-term treatment of histamine-mediated type I hypersensitivity reactions, preferably, selected from acute urticaria. It may also be used in severe cases as additional therapy.

The concept of short-term treatment, associated with a rapid onset of action, is to be understood as a treatment which effect is noticeably within the first 20 minutes after the start of the administration, preferably within the first 15 minutes.

Due to the rapid onset of action of the composition of the invention and very high efficacy, it can be used in severe or acute cases and/or cases where an immediate action is required. The composition is also useful when parenteral administration is necessary or preferred, for example, in uncooperative, nauseous or unconscious patients. Further, due to the prolonged duration of action of the composition of the invention, it can be administered as a once-daily composition.

The term "once daily" is well known to those skilled in the art and intends to mean the administration of a dose to a subject a single time per day, i.e., once approximately every 24 hours. In other words, once daily means that two intravenous, or intramuscular, injections of the pharmaceutical composition must be spaced in time by at least 24 hours.

Therefore, in an embodiment, the invention is directed to the aqueous parenteral pharmaceutical composition of the invention for use in the treatment and/or prevention of an allergic diseases, allergic disorders or allergic symptoms, wherein the composition is administered once a day.

Generally, an effective amount of a compound of the invention will depend on the severity of the disorder being treated and/or prevented and the weight of the sufferer. However, a typical total daily dose may be in the range of from 9.6 mg to 15.0 mg of bilastine.

Therefore, in an embodiment, the invention is directed to the aqueous parenteral pharmaceutical composition of the invention for use in the treatment and/or prevention of an allergic diseases, allergic disorders or allergic symptoms, wherein a total amount of bilastine ranging from 9.6 mg to 15.0 mg is administered per day; preferably wherein a total amount of bilastine ranging from 9.6 mg to 15.0 mg is administered once a day.

In another embodiment, the total daily dose of bilastine may be in the range of from 10.0 mg to 14.0 mg or from 11.0 mg to 13.0 mg.

The parenteral pharmaceutical composition of the invention can be used in the prevention and/or treatment of a subject or patient, wherein the subject or patient is a mammal, preferably a human being, including a human being of any age, race and gender. In an embodiment, the composition of the invention is used as a single therapy for the treatment and/or prevention of the above mentioned diseases, disorders or conditions. That is, bilastine is used as the only active pharmaceutical ingredient in the prevention and/or treatment.

In another embodiment, the composition of the invention is used as an adjunctive treatment, that is in combination with another active pharmaceutical ingredient. In an embodiment, the composition of the invention is used in combination with epinephrine, β₂-agonists (such as salbutamol or albuterol), H₂ antihistamines (such as cimetidine or famotidine), glucocorticoids (such as dexamethasone, methylprednisolone or hydrocortisone), vasopressors (such as norepinephrine (noradrenaline), phenylephrine, dopamine, dobutamine or vasopressin) or glucagon, for example for the treatment of severe diseases, disorders or conditions, such as anaphylaxis.

The pharmaceutical composition of the invention can be administered by parenteral administration. In a particular embodiment, it is administered by intramuscular or intravenous; preferably, intramuscular administration.

The term "treatment" or "to treat" in the context of this specification means administration of a composition according to the invention to ameliorate or eliminate the disease or one or more symptoms associated with said disease. "Treatment" also encompasses ameliorating or eliminating the physiological sequelae of the disease.

The term "ameliorate" in the context of this invention is understood as meaning any improvement on the situation of the patient treated.

The term "prevention" or "to prevent" in the context of this specification means administration of a composition according to the invention to reduce the risk of acquiring or developing the disease or one or more symptoms associated with said disease.

The expression "aqueous parenteral pharmaceutical composition" refers to a liquid pharmaceutical composition comprising water and suitable for parenteral administration.

The expression "therapeutically effective amount" means the amount of a medicament which when administered supplies an amount of one or more pharmaceutically active agents contained therein to provide a therapeutic benefit in the treatment or management of a disease or condition.

In a particular embodiment, the pharmaceutical composition of the invention is not suitable for cosmetic uses, i.e., it is not suitable for a non-therapeutic use.

### Particular embodiments

Particular embodiment 1. Aqueous parenteral pharmaceutical composition comprising:
a) between 0.96% w/v and 2.60% w/v of bilastine, or a pharmaceutically acceptable salt or solvate thereof, and
b) between 10% w/v and 30% w/v of a β-cyclodextrin selected from unmodified β-cyclodextrin, C₁-C₆ alkyl-β-cyclodextrin, C₁-C₆ hydroxyalkyl β-cyclodextrin, C₁-C₆ carboxyalkyl-β-cyclodextrin, carbonyl-β-cyclodextrin, C₁-C₆ sulfoalkylether β-cyclodextrin and mixtures thereof, and
wherein the pH value of the composition is between 3.0 and 7.2, both lower and upper limits of the range included.

Particular embodiment 2. Pharmaceutical composition according to particular embodiment 1, wherein the composition does not comprise a gelling agent.

Particular embodiment 3. Pharmaceutical composition according to any one of particular embodiments 1 to 2, wherein the composition consists of:
a) between 0.96% w/v and 2.60% w/v of bilastine, or a pharmaceutically acceptable salt or solvate thereof,
b) between 10% w/v and 30% w/v of a β-cyclodextrin selected from unmodified β-cyclodextrin, C₁-C₆ alkyl-β-cyclodextrin, C₁-C₆ hydroxyalkyl β-cyclodextrin, C₁-C₆ carboxyalkyl-β-cyclodextrin, carbonyl-β-cyclodextrin, C₁-C₆ sulfoalkylether β-cyclodextrin and mixtures thereof,
c) a pH adjusting agent to provide a pH value between 3.0 and 7.2, both lower and upper limits of the range included,
d) water, and
e) optionally, one or more pharmaceutically acceptable parenteral excipients or carriers selected from preservatives, including antimicrobial preservatives and chemical preservatives, tonicity agents, buffering agents, antioxidants, chelating agents, bulking agents, solubilizing agents, surfactants, co-solvents and mixtures thereof.

Particular embodiment 4. Pharmaceutical composition according to any one of particular embodiments 1 to 3, wherein the composition comprises between 1.0% w/v and 1.4% w/v of bilastine, or a pharmaceutically acceptable salt or solvate thereof.

Particular embodiment 5. Pharmaceutical composition according to any one of particular embodiments 1 to 4, wherein the composition comprises between 1.05% w/v and 1.35% w/v of bilastine, or between 1.10% w/v and 1.30% w/v, or a pharmaceutically acceptable salt or solvate thereof.

Particular embodiment 6. Pharmaceutical composition according to any one of particular embodiments 1 to 5, wherein the composition comprises between 10% w/v and 25% w/v of the β-cyclodextrin.

Particular embodiment 7. Pharmaceutical composition according to any one of particular embodiments 1 to 6, wherein the composition comprises between 12% w/v and 22% w/v, or between 15% w/v and 20% w/v, of the β-cyclodextrin.

Particular embodiment 8. Pharmaceutical composition according to any one of particular embodiments 1 to 7, wherein the β-cyclodextrin is a C₁-C₆ hydroxyalkyl β-cyclodextrin, preferably hydroxypropyl β-cyclodextrin.

Particular embodiment 9. Pharmaceutical composition according to any one of particular embodiments 1 to 8, wherein the pH value of the composition is between 4.0 and 7.0, preferably between 4.0 and 6.0.

Particular embodiment 10. Pharmaceutical composition according to any one of particular embodiments 1 to 9, wherein the composition comprises:
a) between 1.0% w/v and 1.4% w/v of bilastine, or a pharmaceutically acceptable salt or solvate thereof, and
b) between 10% w/v and 25% w/v of a C₁-C₆ hydroxyalkyl β-cyclodextrin, such as hydroxypropyl β-cyclodextrin, and
wherein the pH value of the composition is between 4 and 6, both lower and upper limits of the range included.

Particular embodiment 11. Pharmaceutical composition according to any one of particular embodiments 1 to 10 for use as a medicament.

Particular embodiment 12. Pharmaceutical composition according to any one of particular embodiments 1 to 10 for use in the treatment and/or prevention of an allergic disease or disorder or allergic symptoms.

Particular embodiment 13. Pharmaceutical composition for use according to any one of particular embodiments 11 to 12, for use in the treatment and/or prevention of rhinitis, conjunctivitis, rhinoconjunctivitis, hay fever, dermatitis, eczema, erythema, pruritus, itchiness, urticaria, asthma, anaphylaxis, acute urticaria, chronic urticaria, acute exacerbation of chronic urticaria, immediate-type-hypersensitivity reactions, angioedema, food allergy or drug allergy.

Particular embodiment 14. Pharmaceutical composition for use according to any one of particular embodiments 11 to 13, wherein the composition is administered by intravenous or intramuscular administration.

Particular embodiment 15. Pharmaceutical composition for use according to any one of particular embodiments 11 to 14, wherein the composition is administered once a day.

The following examples are merely illustrative of certain embodiments of the invention and cannot be considered as restricting it in any way.

### EXAMPLES

### Materials and methods

The following materials have been used: bilastine (Faes Farma, batch No. 5000015521); hydroxypropyl-β-cyclodextrin (HPβCD, Kleptose parenteral Roquette Pharma, degree of molar substitution 0.65, molecular weight 1399, batch No. 1605157); HCl37% (VWR Prolabo; batch No. 11E230022); 0.22 µm membrane filters (Millex^{®}-GS, MCE membrane, 33 mm diameter) and 0.45 µm sterilized filters (Biofil^{®}, Nylon membrane, 13mm Ø); 2 mL transparent type II glass ampoules.

The water used in the following examples was purified water obtained using an Elix^{®} water purification system from Merck-Millipore. A WiseStir^{®} Multipoint Magnetic Stirrer was used for the stirring of the solutions.

The pH of the solutions was determined by a Hanna HI5222 pHmeter with a HI1330B micropH electrode.

The concentration of bilastine in the solubility tests was determined by UV/vis spectrophotometer was a Cary^{®} 60 UV-VIS.

The concentration of bilastine in the stability tests was determined by HPLC (Merck Hitachi, Germany) equipped with a diode array detector (L-4500, Merck Hitachi), a compatible pump (L6200 A, Merck Hitachi) and an autosampler (AS4000 A, Merck Hitachi) equipped with a system injection of 100 µl capacity, a control module (Model D-6500, Hitachi) and Model D-7000 HMS version 4.0 data processing software (Merck Hitachi). The chromatographic analysis was carried out with a Waters Symmetry C18 column (3.9 x 150 mm, 5 µm particle size) kept at 30 °C with a column thermostat (L-5025, Merck).

An autoclave from Raypa Steam Sterilizer (Terrassa, Spain) was used to determine the thermostability of the formulations.

The formulation stability was tested in climatic chambers at 5°C ± 3 (brand: ERATIS, model: ICH2000), at 40°C / 75% RH, (brand: INELTEC, model: CCLR-0/1360,) and at 25°C / 60% RH (code CAM027).

### Example 1. Solubility of bilastine vs. HPβCD concentration and pH

The solubility of bilastine was determined at different concentrations of HPβCD and pH values.

The desired amount of HPβCD was dispersed in 40 ml of purified water in a 50 ml flask until complete dissolution and further diluted with water up to a final volume of 50 ml. Each solution was divided into three solutions (15 ml each) and the pH was adjusted with HCl 37% to the desired values (3, 5 or 7, respectively). Each solution was transferred again into three vials (5mL solution each) in order to have several replicates for each sample. Bilastine was added in excess to each vial and the solutions were kept under magnetic stirring for 24 hours. After that time, the pH was adjusted again and they were kept stirring for another 24 hours. Finally, the samples were filtered through 0.45µm filters (Biofil^{®}, Nylon membrane, 13mm Ø), the final pH was checked and the concentration was determined by UV spectrophotometry at a wavelength of 254 nm. The statistical analysis of the results was carried out using the Centurion XVI Statgraphics program version 16.1.15. The results are shown in the following table.

| Entry | HPβCD (% w/v) | Bilastine (mg/mL) | Final pH |
|---|---|---|---|
| 1 | 10 | 18.27 | 4.70 |
| 2 | 20 | 35.11 | 4.60 |
| 3 | 30 | 37.77 | 4.14 |
| 4 | 20 | 18.38 | 5.16 |
| 5 | 30 | 27.22 | 5.13 |
| 6 | 30 | 16.93 | 7.15 |

A very high concentration of bilastine was obtained for the assayed compositions.

### Example 2. Stability of bilastine formulations

Eight batches (denoted as Formulations A-I) with different concentrations of HPβCD and pH adjustments were prepared in order to perform a preliminary stability study at three different conditions (5°C ± 3°C; 25°C / 60% RH; and 40°C / 75% RH) for six months.

Batch A was prepared as follows. 40 ml of purified water were placed into the mixing flask and 10g of HPβCD were added under continuous stirring until complete dissolution. The solution was diluted with purified water up to a final volume of 50 mL. To this solution, 1.2 g of bilastine were added and the solution was stirred for 1 hour. HCl 37% was added until complete solubilization of the Bilastine. Finally, the pH was adjusted to 4 with HCl 37%.

The samples were filtered through 0.22 µm membrane filters (sterilizing filtration, Millex^{®}-GS filters, MCE membrane, 33 mm Ø) and directly placed into the final ampoule (2 ml volume). The process was carried out in a laminar flow hood using sterile material. Finally, samples were taken for analysis.

The same method was used to prepare batches B-I with the composition shown in the following table.

| Formulation | Bilastine (mg/mL) | HPβCD (% w/v) | pH | Purified water |
|---|---|---|---|---|
| A | 24 | 20 | 4 | q.s. 50 mL |
| B | 24 | 20 | 4.5 | q.s. 50 mL |
| C | 24 | 25 | 4 | q.s. 50 mL |
| D | 24 | 25 | 4.5 | q.s. 50 mL |
| E | 24 | 30 | 4 | q.s. 50 mL |
| F | 24 | 30 | 4.5 | q.s. 50 mL |
| G | 12 | 15 | 5.10 | q.s. 50 mL |
| H | 12 | 17 | 5.13 | q.s. 50 mL |
| I | 12 | 19 | 5.5 | q.s. 50 mL |

The preliminary stability study was performed for 6 months, but the formulations were analysed at 0, 1, 2, 3 and 6 months to check the following parameters: macroscopic examination of the packaging, appearance and colour of the solution, presence/absence of visible particles, pH and concentration of bilastine.

For the analysis of pH and bilastine concentration, the ampoules were opened and the content was filtered through sterilized 0.45 µm filters (Biofil^{®}, Nylon membrane, 13 mm Ø). The concentration of bilastine (mg/mL) was determined by HPLC (phase A 58%: acetonitrile/methanol; phase B 42%: 10 mM bicarbonate buffer).

The results of the concentration of bilastine and pH are shown in the following table (only the results at 0, 3 and 6 months are shown).

| | Initial | | | 3 months | | | 6 months | | |
|---|---|---|---|---|---|---|---|---|---|
| | Bilastine concentration (mg/mL) | | | Bilastine concentration (mg/mL) | | | Bilastine concentration (mg/mL) | | |
| Form. | X̅ | SD | pH | X̅ | SD | pH | X̅ | SD | pH |
| A, 5°C | 25.631 | 0.42 | 4.01 | 25.532 | 0.239 | 4.04 | 22.978 | 1.141 | 3.90 |
| A, 25°C | * | * | * | 25.880 | 0.246 | 4.02 | 23.587 | 0.198 | 3.89 |
| A, 40°C | * | * | * | 25.746 | 0.169 | 4.01 | 23.766 | 0.287 | 3.91 |
| B, 5°C | 25.204 | 1.06 | 4.53 | 27.114 | 0.831 | 4.53 | 24.396 | 0.627 | 4.38 |
| B, 25°C | * | * | * | 26.824 | 0.830 | 4.53 | 23.646 | 1.026 | 4.33 |
| B, 40°C | * | * | * | 26.026 | 0.081 | 4.52 | 24.914 | 1.225 | 4.42 |
| C, 5°C | 24.489 | 1.37 | 4.02 | 26.557 | 0.153 | 3.99 | 24.644 | 0.790 | 3.86 |
| C, 25°C | * | * | * | 26.438 | 0.619 | 3.97 | 24.087 | 0.302 | 3.87 |
| C, 40°C | * | * | * | 26.460 | 0.279 | 3.97 | 24.230 | 0.193 | 3.86 |
| D, 5°C | 25.472 | 1.03 | 4.52 | 27.056 | 0.646 | 4.42 | 25.147 | 0.193 | 4.44 |
| D, 25°C | * | * | * | 26.182 | 0.134 | 4.54 | 25.560 | 0.251 | 4.43 |
| D, 40°C | * | * | * | 26.621 | 0.931 | 4.54 | 25.362 | 0.199 | 4.42 |
| E, 5°C | 24.542 | 1.67 | 4.02 | 25.122 | 1.216 | 4.07 | 24.718 | 0.250 | 3.93 |
| E, 25°C | * | * | * | 25.999 | 0.222 | 4.02 | 24.144 | 0.174 | 3.93 |
| E, 40°C | * | * | * | 26.778 | 0.543 | 4.01 | 25.341 | 0.584 | 3.93 |
| F, 5°C | 25.064 | 0.87 | 4.5 | 25.886 | 0.435 | 4.42 | 25.095 | 0.017 | 4.48 |
| F, 25°C | * | * | * | 25.608 | 0.106 | 4.50 | 24.505 | 0.376 | 4.42 |
| F, 40°C | * | * | * | 26.791 | 0.78 | 4.51 | 25.045 | 1.126 | 4.43 |
| G, 5°C | 13.01 | * | 5.10 | * | * | * | 12.256 | 0.268 | 5.18 |
| G, 25°C | * | * | * | * | * | * | 12.131 | 0.045 | 5.09 |
| G, 40°C | * | * | * | * | * | * | 12.311 | 0.187 | 5.06 |
| H, 5°C | 12.44 | * | 4.96 | * | * | * | 12.057 | 0.253 | 5.12 |
| H, 25°C | * | * | * | * | * | * | 11.983 | 0.283 | 5.11 |
| H, 40°C | * | * | * | * | * | * | 12.509 | 0.094 | 5.10 |
| I, 5°C | 13.21 | * | 5.54 | * | * | * | 12.514 | 0.268 | 5.53 |
| I, 25°C | * | * | * | * | * | * | 12.225 | 0.053 | 5.54 |
| I, 40°C | * | * | * | * | * | * | 12.266 | 0.150 | 5.53 |

All the formulations were found to be stable. The best stability results after 6 months were found for Formulation H (12 mg/ml bilastine, 17% HPβCD, pH 5.1) and Formulation D (24 mg/ml bilastine, 25% HPβCD, pH 4.5).

### Example 3. Thermostability study

To verify that sterilization of the samples can be carried out with the terminal sterilization process by autoclave, a new batch of formulation H (12 mg/ml bilastine, 17% HPβCD, pH 5.1) and its placebo (same composition without bilastine) were manufactured. Both formulations were packaged in type I glass transparent ampoules of 2 mL and were sterilized by autoclave at 121°C during 15 min. The samples were analyzed by HPLC. Then, formulation H and its placebo with and without terminal sterilization process were compared.

No differences were found between formulation H and the placebo formulation before and after sterilization process by autoclave. Therefore, the thermostability study shows that terminal sterilization by autoclave does not affect the stability of the formulations.

### Example 4. Preparation of pharmaceutical compositions

The pharmaceutical composition comprising bilastine used in the following examples was prepared as follows.

Bilastine concentration 12 mg/ml (1.2% w/v) and bilastine 0 mg/ml (placebo) batches were manufactured under GMP conditions for investigational medicinal products.

The clinical batch size of bilastine 12 mg/ml parenteral solution and placebo were 80 L for each one.

The manufacturing formula for drug product and placebo are described in the table below.

### Drug product

| Raw material | Unit formula (mg/mL) | Industrial batch (kg/ 80 L) |
|---|---|---|
| Bilastine | 12 | 0.96 |
| Hydroxypropyl β-cyclodextrine | 170 | 13.6 |
| Concentrated hydrochloric acid | q.s. to adjust pH 4.96 | q.s. to adjust pH 4.96 |
| Sterile water for injection | q.s. 1 mL | q.s. 80 L |

### Placebo formula

| Raw material | Unit formula (mg/mL) | Industrial batch (kg/ 80 L) |
|---|---|---|
| Hydroxypropyl β-cyclodextrine | 170 | 13.6 |
| Concentrated hydrochloric acid | q.s. to adjust pH 4.96 | q.s. to adjust pH 4.96 |
| Sterile water for injection | q.s. 1 mL | q.s. 80 L |

The formulations were prepared by adding the raw materials to the reactor previously cleaned and sterilized in the following sequence. 75% of Water for Injection (WFI) was added under stirring and constant stream of nitrogen. Hydroxypropyl β-cyclodextrin was slowly added under stirring for approximately 10 minutes until complete dissolution. Bilastine was slowly added under stirring and constant stream of nitrogen for approximately 30 minutes until complete dissolution. Concentrated hydrochloric acid solution was prepared with Water for Injection (WFI) and the necessary amount was added to adjust the pH of the solution between 4.92-5.00 under stirring for approximately 5-10 minutes until complete dissolution. The pH of the solution (4.92-5.00) was verified. The remainder of the water for injection was added under constant stream of nitrogen and stirring for approximately 20 minutes.

The parenteral composition comprising bilastine was filtered under laminar flow and stream of nitrogen (0.20 µm).

The bilastine parenteral composition was packaged in 2 mL type I glass transparent open ampoules. The ampoules were first subjected to a cleaning and depyrogenation process.

The ampoules were filled with the filtered bilastine composition (18-22 °C), which were then closed and sterilized by autoclave (121 °C, 20 min).

### Example 5. Clinical efficacy of aqueous formulations

This Example, along with Table 1 and Figures 1-5 show the results of a single-dose, five-arm crossover, randomized, double-blind and placebo-controlled study to assess peripheral anti-H₁ activity of bilastine 12 mg intravenous (i.v.) and intramuscular (i.m.) formulation in comparison to Polaramine^{®} 5 mg intramuscular formulation and bilastine 20 mg oral formulation in healthy volunteers.

Twenty-five subjects (10 males and 15 females) aged between 18 to 45 years old at the time of enrolment were randomly distributed to receive:
- *Placebo* (intramuscular) + *Bilaxten*^{®} (oral) + *Placebo* (intravenous)
- *Bilastine* (intramuscular) + *Placebo* (oral) + *Placebo* (intravenous)
- *Placebo* (intramuscular) + *Placebo* (oral) + *Bilastine* (intravenous)
- *Polaramine*^{®} (intramuscular) + *Placebo* (oral) + *Placebo* (intravenous)
- *Placebo* (intramuscular) + *Placebo* (oral) + *Placebo* (intravenous)

The trial comprised five experimental periods of treatment separated by a minimum wash-out period of 7 days. All volunteers received all the treatments. Finally, 23 subjects (9 males and 14 females) completed their participation in the study.

The primary objective of this study was to compare the peripheral anti-H₁ activity of bilastine 12 mg i.v. formulation, bilastine 12 mg i.m. formulation, bilastine 20 mg oral formulation and Polaramine^{®} 5 mg i.m. formulation among them and versus placebo.

The *secondary objectives* of the study were:
· assessing the peripheral anti-H₁ activity (onset of action, maximum effect and maximum effect time) of the treatments;
· evaluating the subjective sensation of itching after histamine inoculation;
· defining the pharmacokinetic profile of the different drugs studied;
· assessing the potential drowsiness/somnolence effects of the treatments;
· assessing the safety and tolerability of the treatments.

The aqueous pharmaceutical compositions of bilastine were prepared at a bilastine concentration of 1.2% w/v and hydroxypropyl β-cyclodextrine concentration of 17% w/v. The pH was adjusted to 4.96 with concentrated HCl. The placebo solution did not contain any bilastine.

### Peripheral anti-H1 activity

Subjects were subjected to a wheal-and-flare test, the most common tool used in human antihistamine pharmacodynamic research. The wheal-and-flare test measures the drug effects in the cutaneous reactivity induced by histamine intradermal injection. The ability for suppression of the histamine-induced wheal and flare in the skin is considered an indicator of antihistaminic potency and consequently, an indicator of potential efficacy. This test relies on the ability of epicutaneously injected histamine to bring about the wheal and flare, a neurovascular response that involves reflex vasodilation (flare) and local swelling caused by plasma extravasation (wheal). The first stage, called the wheal, is controlled by substances called acute phase mediators. The second stage, called the flare, is controlled by substances called late phase mediators. The wheal and flare response is a typical descriptor to characterize a two stage allergic response seen in Type 1 hypersensitivity.

In this study, antihistamine activity was assessed by measurement of the wheal and flare area induced by a histamine skin reaction test performed before and after a single daily bilastine dose administration. Histamine skin reaction tests were performed at the following time points: pre-dose (baseline), +15 min, +30 min, +45 min, +1 h, +2 h, +4 h, +6 h, +9 h, +12 h and +24 h post-drug administration.

This histamine skin reaction test consists in the inoculation of 0.05 ml of a histamine solution of 100 mg/ml in the ventral forearm. Each histamine inoculation was performed into one of six randomly assigned zones on the ventral forearm, alternating arms each time and leaving a minimum distance of 2.0 cm between applications. The surface areas were quantified using Visitrak System (or similar) which automatically completes the area calculations in cm².

In order to assess the peripheral anti-H₁ activity the following parameters were determined:
- Onset of action: the first time point (h) where wheal and flare surface areas show statistically significant differences, compared to their baseline values.
- Maximum effect: maximum percentage of reduction of wheal and flare surface areas.
- Maximum effect time: time point (h) in which the maximum percentage of reduction of wheal and flare surface areas is reached.

Firstly, the % reduction of the wheal area with respect to baseline was studied in 23 healthy subjects and monitored with time from 15 min to 24h (Fig. 2). Both intravenous and intramuscular bilastine showed a significant reduction in the wheal area compared to intramuscular Polaramine^{®} and placebo already after 15 or 30 min, respectively, from the injection. The % reduction peaked at about 2 hours and did plateau until 12 hours, after which the response started decreasing. It is important to note that the effect of i.m. and i.v. bilastine is at least 3 times higher than Polaramine^{®} in reducing the wheal area.

Next, the % reduction of the flare area was also examined compared to the baseline and a significant % reduction is observed in as little as 15 min from the injection of Polaramine^{®}, intramuscular bilastine and intravenous bilastine formulations (Fig. 3). The % reduction peaked at about 2 hours of intravenous and intramuscular bilastine injection and started decreasing again after 12 hours. On the other hand, intramuscular Polaramine^{®} stayed steady along the whole time range. It is important to note that intramuscular and intravenous bilastine caused a reduction of the flare area at least 4 times higher than Polaramine^{®}.

### Subjective sensation of itching

In order to assess the efficacy on itching experienced by the subject, a self-reported visual analogue scale (VAS) on subjective itching sensation was performed after histamine inoculation. Subjective evaluation of itching was performed at the following time points: pre-dose (baseline), +15 min, +30 min, +45 min, +1 h, +2 h, +4 h, +6 h, +9 h, +12 h and +24 h post-drug administration.

Intramuscular bilastine, intravenous bilastine and Polaramine^{®} performed similarly in reducing the itching sensation during the first hour from injection, however intravenous and intramuscular bilastine were about 2 times more effective than Polaramine^{®} in the range from 2 to 6 hours and 2 to 9 hours, respectively (Fig. 4).

### Subjective evaluation of drowsiness/somnolence

Another important aspect of the study is related to the potential side-effects of the parenteral formulations of bilastine. It is known that first-generation antihistaminic may generate substantial drowsiness in patients.

In order to assess the drowsiness/somnolence effects of the study drugs, a self-reported visual analogue scale (VAS) on subjective drowsiness/somnolence effects was performed. Subjective evaluation of drowsiness/somnolence was performed at the following time points: pre-dose (baseline), +1.5 h and +3 h post-drug administration. By means of a visual analogue scale (VAS).

Fig. 5 shows that intramuscular Polaramine^{®} generated at least 8 times more drowsiness - as perceived by the subjects - than intramuscular bilastine at 1.5 hours from the injection, and almost 4 times more drowsiness than intramuscular bilastine at 3 hours from the injection. Intramuscular Polaramine^{®} generated at least 4 times more drowsiness - as perceived by the subjects - than intravenous bilastine at 1.5 hours from the injection, and almost 2 times more drowsiness than intravenous bilastine at 3 hours from the injection. Perhaps, the most interesting fact is that intramuscular and intravenous bilastine appear to be as safe and well-tolerated as the placebo across the whole range of time. In fact, no statistical significant differences between placebo and i.m. bilastine and between placebo and i.v. bilastine were detected (Fig. 5). In contrast, statistical significant differences were found between i.m. Polaramine^{®} and i.m. bilastine and between i.m. Polaramine^{®} and placebo (Fig. 5).

### Pharmacokinetic profile

In order to obtain a correct characterization of the kinetic curve 5 ml blood samples were obtained at different time points.

The following pharmacokinetic parameters were determined: Drug plasma concentration and pharmacokinetic parameters (AUC₀^{t}, AUC₀^{∞}, Cₘₐₓ, tₘₐₓ, t_{1/2}, Kel, Cl, Vd) calculated from those plasma levels by means of Phoenix^{™} WinNonlin^{®} v.8.3.

The data in Table 1 show the pharmacokinetic parameters of the various treatments used in the clinical trial.

**Table 1. Pharmacokinetic data relative to the treatments of the clinical trial.**

| **Variable** | **Units** | **Oral Bilastine** | **i.m. Bilastine** | **i.v. Bilastine** | **i.m. Polaramine** |
|---|---|---|---|---|---|
| **AUC_{ALL}** | min*ng/ml | 63896.6380 | 61506.0467 | 52775.6234 | 10112.8974 |
| **AUC_{INF}** | min*ng/ml | 66333.5734 | 61797.0845 | 53936.5003 | 15560.2592 |
| **CI/F** | ml/min | 329.9916 | 211.8799 | 235.0807 | 370.7845 |
| **C_{MAX}** | ng/ml | 192.7226 | 456.0887 | 1237.6126 | 33.8481 |
| **Lambda_z** | 1/min | 0.0032 | 0.0036 | 0.0034 | 0.0008 |
| **t_{½}** | min | 217.9301 | 195.5388 | 222.1173 | 926.1131 |
| **t_{½}** | h | 3.63 | 3.26 | 3.70 | 15.44 |
| **t_{MAX}** | min | 91.3043 | 39.1304 | 3.9500 | 33.9130 |
| **t_{MAX}** | h | 1.52 | 0.65 | 0.06 | 0.57 |
| **Vz/F** | ml | 104529.3764 | 60128.5403 | 74940.6276 | 444307.9738 |

As it can be appreciated in Table 1 and Fig. 1, bilastine in all pharmaceutical forms and every administration routes (oral, i.m. and i.v.) have a similar AUC (area under the curve), indicating a similar body exposure to the drug. Additionally, C_{MAX} and t_{MAX} of bilastine varies between the three formulations depending on the administration route, with a much higher C_{MAX} and lower t_{MAX} for i.m. and i.v. bilastine compared to its oral administration. Furthermore, the elimination half-life (t_{½}) remains very similar and stable between all pharmaceutical forms and administration routes, indicating that bilastine is very stable in every form.

Finally, Polaramine^{®} shows a t_{MAX} very similar to i.m. bilastine, indicating that the i.m. absorption is alike.

## Claims

1. Aqueous parenteral pharmaceutical composition comprising:
a) between 0.96% w/v and 2.60% w/v of bilastine, or a pharmaceutically acceptable salt or solvate thereof, and
b) between 10% w/v and 30% w/v of a β-cyclodextrin selected from unmodified β-cyclodextrin, C₁-C₆ alkyl-β-cyclodextrin, C₁-C₆ hydroxyalkyl β-cyclodextrin, C₁-C₆ carboxyalkyl-β-cyclodextrin, carbonyl-β-cyclodextrin, C₁-C₆ sulfoalkylether β-cyclodextrin and mixtures thereof, and
wherein the pH value of the composition is between 3.0 and 7.2, both lower and upper limits of the range included, and wherein parenteral is selected from intravenous or intramuscular.

2. Pharmaceutical composition according to claim 1, wherein the composition does not comprise a gelling agent.

3. Pharmaceutical composition according to any one of claims 1 to 2, wherein the composition consists of:
a) between 0.96% w/v and 2.60% w/v of bilastine, or a pharmaceutically acceptable salt or solvate thereof,
b) between 10% w/v and 30% w/v of a β-cyclodextrin selected from unmodified β-cyclodextrin, C₁-C₆ alkyl-β-cyclodextrin, C₁-C₆ hydroxyalkyl β-cyclodextrin, C₁-C₆ carboxyalkyl-β-cyclodextrin, carbonyl-β-cyclodextrin, C₁-C₆ sulfoalkylether β-cyclodextrin and mixtures thereof,
c) a pH adjusting agent to provide a pH value between 3.0 and 7.2, both lower and upper limits of the range included,
d) water for injection, and
e) optionally, one or more pharmaceutically acceptable parenteral excipients or carriers selected from preservatives, including antimicrobial preservatives and chemical preservatives, tonicity agents, buffering agents, antioxidants, chelating agents, bulking agents, solubilizing agents, surfactants, co-solvents and mixtures thereof.

4. Pharmaceutical composition according to any one of claims 1 to 3, wherein the composition comprises between 1.0% w/v and 1.4% w/v of bilastine, preferably between 1.1% w/v and 1.3% w/v of bilastine, or a pharmaceutically acceptable salt or solvate thereof.

5. Pharmaceutical composition according to any one of claims 1 to 4, wherein the composition comprises between 10% w/v and 25% w/v of the β-cyclodextrin, preferably between 15% w/v and 20% w/v of the β-cyclodextrin.

6. Pharmaceutical composition according to any one of claims 1 to 5, wherein the β-cyclodextrin is a C₁-C₆ hydroxyalkyl β-cyclodextrin, preferably hydroxypropyl β-cyclodextrin.

7. Pharmaceutical composition according to any one of claims 1 to 6, wherein the pH value of the composition is between 4.0 and 7.0, preferably between 4.0 and 6.0.

8. Pharmaceutical composition according to any one of claims 1 to 7, wherein the composition comprises:
a) between 1.0% w/v and 1.4% w/v of bilastine, or a pharmaceutically acceptable salt or solvate thereof, and
b) between 10% w/v and 25% w/v of a C₁-C₆ hydroxyalkyl β-cyclodextrin, such as hydroxypropyl β-cyclodextrin, and
wherein the pH value of the composition is between 4 and 6, both lower and upper limits of the range included.

9. Pharmaceutical composition according to any one of claims 1 to 8, wherein the volume of each unit dose is from 0.5 to 1.5 mL, preferably from 0.8 to 1.2 mL.

10. Pharmaceutical composition according to any one of claims 1 to 9, wherein the composition is a once-daily composition.

11. Pharmaceutical composition according to any one of claims 1 to 10 for use as a medicament.

12. Pharmaceutical composition according to claim 11, for use in the treatment and/or prevention of an allergic disease or disorder or allergic symptoms.

13. Pharmaceutical composition according to claim 11, for use in the treatment and/or prevention of acute type I hypersensitivity reactions.

14. Pharmaceutical composition for use according to claim 12, wherein the allergic disease or disorder or allergic symptom is selected from rhinitis, conjunctivitis, rhinoconjunctivitis, hay fever, dermatitis, eczema, erythema, pruritus, itchiness, urticaria, asthma, anaphylaxis, acute urticaria, chronic urticaria, acute exacerbation of chronic urticaria, immediate-type-hypersensitivity reactions, angioedema, food allergy or drug allergy.

15. Pharmaceutical composition for use according to any one of claims 11 to 14, wherein the composition is administered once a day.

## Patentansprüche

1. Wässrige parenterale pharmazeutische Zusammensetzung, umfassend:
a) zwischen 0,96 % w/v und 2,60 % w/v Bilastin, oder ein pharmazeutisch verträgliches Salz oder Solvat davon, und
b) zwischen 10 % w/v und 30 % w/v eines β-Cyclodextrins, ausgewählt aus unmodifiziertem β-Cyclodextrin, C₁-C₆-Alkyl-β-cyclodextrin, C₁-C₆-Hydroxyalkyl-β-cyclodextrin, C₁-C₆-Carboxyalkyl-β-cyclodextrin, Carbonyl-β-cyclodextrin, C₁-C₆-Sulfoalkylether-β-cyclodextrin und Gemischen davon, und
wobei der pH-Wert der Zusammensetzung zwischen 3,0 und 7,2 liegt, wobei sowohl die Unter- als auch die Obergrenze des Bereichs eingeschlossen sind, und wobei parenteral aus intravenös oder intramuskulär ausgewählt ist.

2. Pharmazeutische Zusammensetzung nach Anspruch 1, wobei die Zusammensetzung kein Geliermittel enthält.

3. Pharmazeutische Zusammensetzung nach einem der Ansprüche 1 bis 2, wobei die Zusammensetzung besteht aus:
a) zwischen 0,96 % w/v und 2,60 % w/v Bilastin, oder einem pharmazeutisch verträglichen Salz oder Solvat davon,
b) zwischen 10 % w/v und 30 % w/v eines β-Cyclodextrins, ausgewählt aus unmodifiziertem β-Cyclodextrin, C₁-C₆-Alkyl-β-cyclodextrin, C₁-C₆-Hydroxyalkyl-β-cyclodextrin, C₁-C₆-Carboxyalkyl-β-cyclodextrin, Carbonyl-β-cyclodextrin, C₁-C₆-Sulfoalkylether-β-cyclodextrin und Gemischen davon,
c) einem pH-Einstellmittel, um einen pH-Wert zwischen 3,0 und 7,2 bereitzustellen, wobei sowohl die Unter- als auch die Obergrenze des Bereichs eingeschlossen sind,
d) Wasser zur Injektion und
e) gegebenenfalls einem oder mehreren pharmazeutisch verträglichen parenteralen Exzipienten oder Trägern, ausgewählt aus Konservierungsmitteln, einschließlich antimikrobieller Konservierungsmittel und chemischer Konservierungsmittel, Tonizitätsmitteln, Puffermitteln, Antioxidantien, Chelatbildnern, Füllmitteln, Solubilisierungsmitteln, Tensiden, Co-Lösungsmitteln und Gemischen davon.

4. Pharmazeutische Zusammensetzung nach einem der Ansprüche 1 bis 3, wobei die Zusammensetzung zwischen 1,0 % w/v und 1,4 % w/v Bilastin, vorzugsweise zwischen 1,1 % w/v und 1,3 % w/v Bilastin, oder ein pharmazeutisch verträgliches Salz oder Solvat davon, umfasst.

5. Pharmazeutische Zusammensetzung nach einem der Ansprüche 1 bis 4, wobei die Zusammensetzung zwischen 10 % w/v und 25 % w/v des β-Cyclodextrins, vorzugsweise zwischen 15 % w/v und 20 % w/v des β-Cyclodextrins, umfasst.

6. Pharmazeutische Zusammensetzung nach einem der Ansprüche 1 bis 5, wobei das β-Cyclodextrin ein C₁-C₆-Hydroxyalkyl-β-cyclodextrin, vorzugsweise Hydroxypropyl-β-cyclodextrin, ist.

7. Pharmazeutische Zusammensetzung nach einem der Ansprüche 1 bis 6, wobei der pH-Wert der Zusammensetzung zwischen 4,0 und 7,0, vorzugsweise zwischen 4,0 und 6,0 liegt.

8. Pharmazeutische Zusammensetzung nach einem der Ansprüche 1 bis 7, wobei die Zusammensetzung umfasst:
a) zwischen 1,0 % w/v und 1,4 % w/v Bilastin, oder ein pharmazeutisch verträgliches Salz oder Solvat davon, und
b) zwischen 10 % w/v und 25 % w/v eines C₁-C₆-Hydroxyalkyl-β-cyclodextrins, wie Hydroxypropyl-β-cyclodextrin, und
wobei der pH-Wert der Zusammensetzung zwischen 4 und 6 liegt, wobei sowohl die Unter- als auch die Obergrenze des Bereichs eingeschlossen sind.

9. Pharmazeutische Zusammensetzung nach einem der Ansprüche 1 bis 8, wobei das Volumen jeder Einheitsdosis von 0,5 bis 1,5 ml, vorzugsweise von 0,8 bis 1,2 ml, beträgt.

10. Pharmazeutische Zusammensetzung nach einem der Ansprüche 1 bis 9, wobei die Zusammensetzung eine einmal täglich zu verabreichende Zusammensetzung ist.

11. Pharmazeutische Zusammensetzung nach einem der Ansprüche 1 bis 10 zur Verwendung als Medikament.

12. Pharmazeutische Zusammensetzung nach Anspruch 11 zur Verwendung bei der Behandlung und/oder Vorbeugung einer allergischen Erkrankung oder Störung oder allergischer Symptome.

13. Pharmazeutische Zusammensetzung nach Anspruch 11 zur Verwendung bei der Behandlung und/oder Vorbeugung von akuten Typ-I-Hypersensitivitätsreaktionen.

14. Pharmazeutische Zusammensetzung zur Verwendung nach Anspruch 12, wobei die allergische Erkrankung oder Störung oder das allergische Symptom ausgewählt ist aus Rhinitis, Konjunktivitis, Rhinokonjunktivitis, Heuschnupfen, Dermatitis, Ekzem, Erythem, Pruritus, Juckreiz, Urtikaria, Asthma, Anaphylaxie, akuter Urtikaria, chronischer Urtikaria, akuter Exazerbation einer chronischen Urtikaria, Hypersensitivitätsreaktionen vom Soforttyp, Angioödem, Nahrungsmittelallergie oder Arzneimittelallergie.

15. Pharmazeutische Zusammensetzung zur Verwendung nach einem der Ansprüche 11 bis 14, wobei die Zusammensetzung einmal täglich verabreicht wird.

## Revendications

1. Composition pharmaceutique aqueuse parentérale comprenant :
a) entre 0,96 % p/V et 2,60 % p/V de bilastine, ou un sel ou solvate pharmaceutiquement acceptable de celle-ci, et
b) entre 10 % p/V et 30 % p/V d'une β-cyclodextrine choisie parmi la β-cyclodextrine non modifiée, la C₁-C₆ alkyl-β-cyclodextrine, la C₁-C₆ hydroxyalkyl β-cyclodextrine, la C₁-C₆ carboxyalkyl-β-cyclodextrine, la carbonyl-β-cyclodextrine, la C₁-C₆ sulfoalkyléther β-cyclodextrine et leurs mélanges, et
dans laquelle la valeur du pH de la composition est comprise entre 3,0 et 7,2, les limites inférieure et supérieure de l'intervalle étant incluses, et dans laquelle l'administration parentérale est choisie parmi les voies intraveineuse ou intramusculaire.

2. Composition pharmaceutique selon la revendication 1, dans laquelle la composition ne comprend pas d'agent gélifiant.

3. Composition pharmaceutique selon l'une quelconque des revendications 1 à 2, dans laquelle la composition consiste en :
a) entre 0,96 % p/V et 2,60 % p/V de bilastine, ou un sel ou solvate pharmaceutiquement acceptable de celle-ci,
b) entre 10 % p/V et 30 % p/V d'une β-cyclodextrine choisie parmi la β-cyclodextrine non modifiée, la C₁-C₆ alkyl-β-cyclodextrine, la C₁-C₆ hydroxyalkyl β-cyclodextrine, la C₁-C₆ carboxyalkyl-β-cyclodextrine, la carbonyl-β-cyclodextrine, la C₁-C₆ sulfoalkyléther β-cyclodextrine et leurs mélanges,
c) un agent régulateur de pH pour obtenir une valeur de pH comprise entre 3,0 et 7,2, les limites inférieure et supérieure de l'intervalle étant incluses,
d) de l'eau pour injection, et
e) éventuellement, un ou plusieurs excipients ou supports parentéraux pharmaceutiquement acceptables choisis parmi les conservateurs, y compris les conservateurs antimicrobiens et les conservateurs chimiques, les agents de tonicité, les agents tampons, les antioxydants, les agents chélateurs, les agents de charge, les agents solubilisants, les tensioactifs, les co-solvants et leurs mélanges.

4. Composition pharmaceutique selon l'une quelconque des revendications 1 à 3, dans laquelle la composition comprend entre 1,0 % p/V et 1,4 % p/V de bilastine, de préférence entre 1,1 % p/V et 1,3 % p/V de bilastine, ou un sel ou solvate pharmaceutiquement acceptable de celle-ci.

5. Composition pharmaceutique selon l'une quelconque des revendications 1 à 4, dans laquelle la composition comprend entre 10 % p/V et 25 % p/V de la β-cyclodextrine, de préférence entre 15 % p/V et 20 % p/V de la β-cyclodextrine.

6. Composition pharmaceutique selon l'une quelconque des revendications 1 à 5, dans laquelle la β-cyclodextrine est une C₁-C₆ hydroxyalkyl β-cyclodextrine, de préférence l'hydroxypropyl β-cyclodextrine.

7. Composition pharmaceutique selon l'une quelconque des revendications 1 à 6, dans laquelle la valeur du pH de la composition est comprise entre 4,0 et 7,0, de préférence entre 4,0 et 6,0.

8. Composition pharmaceutique selon l'une quelconque des revendications 1 à 7, dans laquelle la composition comprend :
a) entre 1,0 % p/V et 1,4 % p/V de bilastine, ou un sel ou solvate pharmaceutiquement acceptable de celle-ci, et
b) entre 10 % p/V et 25 % p/V d'une C₁-C₆ hydroxyalkyl β-cyclodextrine, telle que l'hydroxypropyl β-cyclodextrine, et
dans laquelle la valeur du pH de la composition est comprise entre 4 et 6, les limites inférieure et supérieure de l'intervalle étant incluses.

9. Composition pharmaceutique selon l'une quelconque des revendications 1 à 8, dans laquelle le volume de chaque dose unitaire est compris entre 0,5 et 1,5 ml, de préférence entre 0,8 et 1,2 ml.

10. Composition pharmaceutique selon l'une quelconque des revendications 1 à 9, dans laquelle la composition est une composition à prise unique quotidienne.

11. Composition pharmaceutique selon l'une quelconque des revendications 1 à 10, pour une utilisation comme médicament.

12. Composition pharmaceutique selon la revendication 11, pour une utilisation dans le traitement et/ou la prévention d'une maladie ou d'un trouble allergique ou de symptômes allergiques.

13. Composition pharmaceutique selon la revendication 11, pour une utilisation dans le traitement et/ou la prévention des réactions d'hypersensibilité aiguë de type I.

14. Composition pharmaceutique pour une utilisation selon la revendication 12, dans laquelle la maladie ou le trouble allergique ou le symptôme allergique est choisi parmi la rhinite, la conjonctivite, la rhinoconjonctivite, le rhume des foins, la dermatite, l'eczéma, l'érythème, le prurit, les démangeaisons, urticaire, l'asthme, l'anaphylaxie, l'urticaire aiguë, l'urticaire chronique, l'exacerbation aiguë de l'urticaire chronique, les réactions d'hypersensibilité de type immédiat, l'œdème de Quincke, l'allergie alimentaire ou l'allergie médicamenteuse.

15. Composition pharmaceutique pour une utilisation selon l'une quelconque des revendications 11 à 14, dans laquelle la composition est administrée une fois par jour.
